# EUROPEAN PATENT APPLICATION

(11) **EP 1 522 306 A1**
(43) Date of publication of application: **13.04.2005**
(21) Application number: 03103726.0
(22) Date of filing: 08.10.2003
(51) Int. Cl.: A61K 31/567, A61K 31/565, A61P 15/12

(54) **A pharmaceutical product for hormone replacement therapy comprising tibolone or a derivative thereof and estradiol or a derivative thereof**

(71) Applicant: Liconsa, Liberacion Controlada de Sustancias Activas, S.A., 08028 Barcelona (ES)
(72) Inventor: Palacios, Santiago, 28001 Madrid (ES); Perez, Francisco, 08970 Sant Joan Despi (Barcelona) (ES); Banado M., Carlos, 28033 Madrid (ES)
(74) Representative: Markvardsen, Peter

(57) **Abstract**

A pharmaceutical product comprising an effective amount of tibolone or derivative thereof, an effective amount of estradiol or derivative thereof and a pharmaceutically acceptable carrier, wherein the product is provided as a combined preparation for simultaneous, separate or sequential use in a method for hormone replacement therapy or prevention of hypoestrogenism associated clinical symptoms in a human person, in particular wherein the human is a postmenopausal woman.

## Description

### FIELD OF THE INVENTION:

The present invention relates to a pharmaceutical product comprising an effective amount of tibolone or derivative thereof, an effective amount of estradiol or derivative thereof and a pharmaceutically acceptable carrier, wherein the product is provided as a combined preparation for simultaneous, separate or sequential use in a method for hormone replacement therapy or prevention of hypoestrogenism associated clinical symptoms in a human person, in particular wherein the human is a postmenopausal woman.

### BACKGROUND OF THE INVENTION:

In hormone replacement therapy (HRT), sometimes also referred to as estrogen replacement therapy, estrogens are administered to prevent or treat symptoms resulting from estrogen deficiency or hypoestrogenism. Hypoestrogenism can occur in both females and males, and can lead to disorders and ailments such as osteoporosis (loss of bone mass), arteriosclerosis, climacteric symptoms such as hot flushes (flashes), sweats, urogenital atrophy, mood disturbances, insomnia, palpitations. Estrogen deficiency has also been associated with cognitive disturbances and Alzheimer's disease.

Hypoestrogenism, and in particular chronic hypoestrogenism, is frequently observed in perimenopausal and post-menopausal women. However, it can also result from hypogonadism or castration, as well as from primary ovarian failure, treatment of e.g. breast cancer with aromatase inhibitor and gonadotropin-releasing hormone analogue treatment of benign gynaecological diseases such as endometriosis, adenomyosis, uterine fibroids (leiomyomas), dysmenorrlioea, menorrhagia and metrorrhagia.

Hormone replacement therapy (HRT) has been used for many years for alleviation of hypoestrogenism associated clinical symptoms such as postmenopausal symptoms as well as prophylaxis of osteoporosis in postmenopausal women.

Suitable estrogens, such as estradiol, have been used in HRT. Estradiol is many times used in combination with some progestins such as norethindrone (a synthetic progestin also termed norethisterone), medroxiprogesterone and others.

Tibolone is a synthetic steroid which elicits estrogenic, progestogenic and androgenic properties. Tibolone is used as an alternative to estradiol in HRT.

Numerous studies have been carried out to compare the effectiveness of tibolone and estradiol on symptoms in postmenopausal women. Generally, all these studies are based on that a group of women gets tibolone and another group gets estradiol or estradiol plus a progestin such as norethindrone.

The review article of Journal of Steroid Biochemistry and Molecular Biology (2001), 76(1-5), 231-238 provides a review of some of these comparative studies. It further concludes that tibolone works on the estradiol receptor. In other words, tibolone and estradiol are both working on the same receptor.

WO0074684A1 describes numerous different possible combinations of different steroid hormones for treatment of postmenopausal women. Virtually all possible theoretical combinations are listed including different combinations of estrogens, androgens, progestins, selective estrogen receptor modulator (SERM), selective androgen receptor modulator (SARM) and selective progestin receptor modulator (SPRM). No specific relevant actual example results are provided. One list of the description is a list of a number of different suitable SERM molecules. This list includes tibolone. Another list includes a number of different suitable estrogens. This list includes estradiol.

The article of European Journal of Gynaecological Oncology, (2002) 23 (2) 127-30 describes a study analyzing possible breast cancer risks of tibolone. The studies are performed in vitro on a human breast cancer cell line. The aim of the study was to compare tibolone to HRT using estradiol and norethisterone. Tibolone was examined alone and in the presence of 0.1 nM estradiol.

### SUMMARY OF THE INVENTION:

The problem to be solved by the present invention is to provide a pharmaceutical composition with improved properties in relation to treatment or prevention of hypoestrogenism associated clinical symptoms, in particular of a postmenopausal woman. Improved properties include less unwanted negative side effects such as less risk for breast cancer development.

As explained above, the prior art describes that tibolone and estradiol are both working on the same receptor. Among others, despite of this fact, the present inventors investigated the effects of a pharmaceutical product combining both tibolone and estradiol. Surprisingly, they found that such a pharmaceutical product had improved properties in relation to treatment or prevention of hypoestrogenism associated clinical symptoms, in particular of a postmenopausal woman.

Accordingly, a first aspect of the invention relates to a pharmaceutical product comprising an effective amount of tibolone or derivative thereof, an effective amount of estradiol or derivative thereof and a pharmaceutically acceptable carrier, wherein the product is provided as a combined preparation for simultaneous, separate or sequential use in a method for hormone replacement therapy or prevention of hypoestrogenism associated clinical symptoms in a human person, in particular wherein the human is a postmenopausal woman.

A second aspect of the invention relates to use of a pharmaceutical product, as described herein, for the manufacture of a medicament for hormone replacement therapy or prevention of hypoestrogenism associated clinical symptoms in a human person, in particular wherein the human is a postmenopausal woman.

This second aspect may alternatively be formulated as a method for hormone replacement therapy or prevention of hypoestrogenism associated clinical symptoms in a human, in particular wherein the human is a postmenopausal woman, comprising administering to a human in need thereof an effective amount of pharmaceutical product as described herein.

A third aspect of the invention relates to a method for making a pharmaceutical product for use in a method for hormone replacement therapy or prevention of hypoestrogenism associated clinical symptoms in a human person, in particular wherein the human is a postmenopausal woman, comprising making a pharmaceutical product comprising an effective amount of tibolone or derivative thereof, an effective amount of estradiol or derivative thereof and a pharmaceutically acceptable carrier as described herein.

### Embodiment(s) of the present invention is described below, by way of example(s) only

### DETAILED DESCRIPTION OF THE INVENTION:

### Tibolone:

Tibolone denotes herein the molecule with Chemical Abstract Service (CAS) name (7α,17α)-17-hydroxy-7-methyl-19-norpregn-5(10)-en-20-yn-3-one and CAS registry number 5630-53-5.

In vivo, tibolone is rapidly converted by the intestine and liver into two estrogenic metabolites, 3α-hydroxytibolone and 3 β-hydroxytibolone, which are responsible for the drug's estrogenic effects on bone, vaginal tissue and climacteric symptoms. A third metabolite, the Δ4-isomer, has progestagenic and androgenic activities and is specifically formed in the endometrium. For further details see the review article of Journal of Steroid Biochemistry and Molecular Biology (2001), 76(1-5), 231-238.

These tibolone metabolites shall herein be understood to be a derivative of tibolone.

### Estradiol:

Estradiol denotes herein the molecule with Chemical Abstract Service (CAS) name (17β)-Estra-1,3,5(10)-triene-3,17-diol; β-estradiol. The CAS registry number is 50-28-2.

Derivatives of estradiol include closely related molecules such as estradiol valerate, estradiol benzoate, α-estradiol or estradiol 17β-Cypionate.

### Derivatives:

This section is a general description of derivatives. Tibolone and estradiol are active components.

The active components may be converted into derivatives thereof, like esters and ethers, which, within a pharmaceutical composition as described herein, are all covered by the present invention. The derivatives can be prepared by standard procedures known to one skilled in the art.

Esters and ethers may e.g. be prepared by the methods given in the literature, for example, in Advanced Organic Synthesis, 4^{th} Edition, J. March, John Wiley & Sons., 1992.

Generally, a derivative of tibolone denotes herein a derivative that at least is capable of triggering a response in vivo that in essence corresponds to the response in vivo of tibolone. In order for tibolone to trigger such a response tibolone normally have to bind to tibolone receptors, which receptors are found in various tissues within the human body. A derivative of tibolone denotes herein a derivative that is capable of binding to a tibolone receptor in a similar way as tibolone bind to the same tibolone receptor. Based on e.g. known 3-dimensional structure of tibolone and a tibolone receptor the skilled person is perfectly capable of making e.g. minor structural modifications of tibolone to get a derivative of tibolone that fulfills the criteria above.

A derivative of estradiol should herein be understood in the same way, wherein a derivative of estradiol, of course, has a response in vivo that is corresponding to estradiol and a receptor binding that is similar to the binding estradiol has to the same estradiol receptor.

### Pharmaceutical product:

The pharmaceutical product may be provided as
- first container comprising a composition comprising an effective amount of tibolone or derivative thereof and a pharmaceutically acceptable carrier;
- second container comprising a composition comprising an effective amount of estradiol or derivative thereof and a pharmaceutically acceptable carrier; and
- instructions for combined sequential and/or simultaneous administration of the compositions of first and second container for simultaneous or sequential use in a method for hormone replacement therapy or prevention of hypoestrogenism associated clinical symptoms in a human person.

The term "container" should be understood broadly as some kind of physical item. It could e.g. be a tablet, an injector, a capsule etc.

Alternatively, the pharmaceutical product may be a pharmaceutical composition comprising both tibolone and estradiol. Accordingly, an embodiment of the invention relates to a pharmaceutical composition comprising an effective amount of tibolone or derivative thereof, an effective amount of estradiol or derivative thereof and a pharmaceutically acceptable carrier for use in a method for hormone replacement therapy or prevention of hypoestrogenism associated clinical symptoms in a human person.

Preferably, the composition comprises from 1 µg to 10 mg of tibolone or derivative thereof, more preferably from 50 µg to 5mg of tibolone or derivative thereof.

Preferably, the composition comprises from 1 µg to 10 mg of estradiol or derivative thereof, more preferably from 50 µg to 5mg of estradiol or derivative thereof.

By "pharmaceutically acceptable carrier" as used herein is meant one or more compatible solid or liquid filler diluents, or encapsulating substances. By "compatible" as used herein is meant that the components of the composition are capable of being commingled without interacting in a manner which would substantially decrease the pharmaceutical efficacy of the total composition under ordinary use situations.

Some examples of substances which can serve as pharmaceutical carriers are sugars, such as lactose, glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; water; pyrogen-free water; isotonic saline; and phosphate buffer solution; skim milk powder; as well as other non-toxic compatible substances used in pharmaceutical formulations.

Wetting agents, fillers and lubricants such as sodium lauryl sulfate, as well as coloring agents, flavoring agents, lubricants, excipients, tabletting agents, stabilizers, anti-oxidants and preservatives, can also be present.

A pharmaceutical product as described herein can be administered orally, intramuscularly, intravenously, transdermally, subcutaneously or by other modes of administration. Preferably, the pharmaceutical product can be administered orally.

A pharmaceutical product, as described herein, may include other pharmaceutically active substances. It can be prepared by mixing the active compounds with one or more pharmacologically tolerated auxiliaries and/or excipients such as, for example, fillers, emulsifiers, lubricants, masking flavors, colorants, or buffer substances, and converting the mixture into a suitable pharmaceutical form such as, for example, tablets, coated tablets, capsules, granules, powders, emulsions, suspensions, or solutions.

Without being limited to theory, it is believed that tibolone, among others, provides the effect of a progestin. Accordingly, in the pharmaceutical product it is not considered necessary to include progestins such as norethindrone (a synthetic progestin, also termed norethisterone) or medroxiprogesterone. Accordingly, a preferred embodiment of the invention relates to a pharmaceutical product, as described herein, that does not comprise norethindrone or medroxiprogesterone.

Examples of auxiliaries and/or excipients which may be mentioned are tragacanth, lactose, talc, agar, polyglycols, ethanol, and water. It is also possible to administer the active substances as such, without vehicles or diluents, in a suitable form, for example, in capsules.

Preferably, the pharmaceutical product, as described herein, is provided together with suitable pharmaceutically relevant instructions. The instructions preferably explain pharmaceutically relevant information such as e.g. qualitative and quantitative of composition, pharmaceutical form, therapeutic indications and method of administration (including recommend doses).

### Use for the treatment of clinical symptoms:

The HRT method according to the invention may advantageously be used to treat all known forms of hypoestrogenism, e. g. hypoestrogenism associated with peri-menopausal and post-menopausal women, hypoestrogenism resulting from hypogonadism or castration, as well as hypoestrogenism resulting from primary ovarian failure, treatment of e. g. breast cancer with aromatase inhibitor and gonadotropin-releasing hormone analogue treatment of e. g. benign gynaecological diseases. Examples of manifestations of hypoestrogenism that can effectively be treated or prevented with the present method in both females and males include osteoporosis, arteriosclerosis, cognitive disturbances and Alzheimer's disease. The method may also advantageously be used in the (prophylactic) treatment of climacteric symptoms such as hot flushes (flashes), sweats, urogenital atrophy, mood disturbances, insomnia and palpitations. The present method is particularly suited for treating or preventing osteoporosis and climacteric symptoms.

Virtually all postmenopausal and perimenopausal women can be treated with the methods of the invention. If desired, such a woman can be identified as being in need of hormone replacement therapy (using standard criteria, as described, for example, by the American College of Physicians Guidelines (incorporated herein by reference)) prior to treatment of the woman with the pharmaceutical product as described herein. A variety of therapeutic regimens are suitable for use in the invention, and practitioners of ordinary skill in the art can readily optimize a particular regimen for a particular woman by monitoring the woman for signs and symptoms of hormone deficiency, and increasing or decreasing the dosage and/or frequency of treatment as desired.

Preferably, the human to be treated is a postmenopausal woman.

The term a "postmenopausal" woman is one who in the absence of hormone replacement therapy or other medication would experience at least 12 months of amenorrhea or levels of serum follicle-stimulating hormone greater than 30 mIU/ml. Menopause is the period of natural cessation of menstruation occurring usually between the ages of 45 and 50. Accordingly, a postmenopausal woman may alternatively be defined as a woman that has had the menopause.

A "perimenopausal"woman is one who in the absence of hormone replacement therapy or other medication would experience a change in her intermenstrual cycle interval and have associated symptoms of estrogen deficiency, such as vasomotor flushes, vaginal dryness, and worsening premenstrual syndrome. Also included are women who in the absence of hormone replacement therapy or other medication would experience less than 12 months of amenorrhea.

Regardless of the route of administration, the estrogen typically is administered at a dosage of 1 µg/kg to 10 mg/kg (e.g., 5 µg/kg to 5mg/kg). The tibolone is typically is administered at a dosage of 1 µg/kg to 10 mg/kg (e.g., 5 µg/kg to 5mg/kg).

The pharmaceutical product can be administered in multiple doses per day, if desired, to achieve the total desired daily dose. Typically, the human will be treated over the course of several months or years, or even life-long to ameliorate the signs and symptoms.

In a typical therapeutic regimen, the pharmaceutical product is administered to the human at least once daily (e.g., orally, subcutaneously, or delivered by transdermal or depot methods) for at least days, at the dosages listed above. Preferably the pharmaceutical product is administered orally to the human.

Examples of clinical symptoms of a post menopausal woman are osteoporosis, increased cholesterol levels that leads to atherosclerosis, depression, headaches, nausea or climacteric symptoms.

### A separate aspect of the invention:

Without being limited to theory, it is believed that the present invention works in a beneficial manner not only with estradiol and its derivatives but with estrogens in general.

Accordingly a separate aspect of the invention relates to a pharmaceutical product comprising an effective amount of tibolone or derivative thereof, an effective amount of an estrogen or derivative thereof and a pharmaceutically acceptable carrier, wherein the product is provided as a combined preparation for simultaneous, separate or sequential use in a method for hormone replacement therapy or prevention of hypoestrogenism associated clinical symptoms in a human person.

All other aspects and embodiments described herein with respect to a pharmaceutical product and its related uses and method for making it are also aspects and preferred embodiments with respect to this separate aspect of the invention.

An "estrogen" is an agent, natural or synthetic, that exerts biological effects characteristic of estrogenic hormones such as estradiol. As used herein, the term "estrogen" also encompasses "conjugated estrogens," which are an amorphous preparation of naturally occurring, water soluble, conjugated forms of mixed estrogens that typically are obtained from the urine of pregnant mares (e.g., sodium estrone sulfate).

Examples of suitable estrogens include, without limitation, estradiol valerate, estradiol benzoate, estradiol, estradiol cypionate, estrone, piperazine estrone sulfate, estriol, estriol hemisuccinate, ethinylestradiol, polyestradiol phosphate, estrone potassium sulfate, benzestrol, chlorotrianisene, methallenestril, dienestrol, diethylstilbestrol diphosphate, mestranol, diethylestilbestrol (DES), quinestranol, phytoestrogens, animal-derived estrogens (e.g., equine estrogens), and metabolic derivatives of animal-derived estrogens.

### EXAMPLES:

### Example 1: Combined estrogen/tibolone treatment

### Introduction:

The principal objective of this study was to investigate the possibility of utilizing tibolone as a progestin in a combined estrogen/tibolone treatment of menopausal problems.

### Material and methods

112 Wistar female rats were used. 96 were castrated at 3 month age. The rats were split into following experimental groups (n=16): The rats of groups A to F were castrated. Group G was not castrated. All doses were given via subcutaneous (SC) route.
Group A: received estradiol valerate (E2) at a dose of 50 µg/Kg/week;
Group B: received tibolone at a dose of 1 mg/Kg/day;
Group C: received a combination of E2 and tibolone at the same doses as given for group A and B;
Group D: was as group C but only with a tibolone dose of 0.5 mg/Kg/day;
Group E: received E2 together with noretisterone at a dose of 0.5 mg/Kg/day;
Group F: was a control group of castrated rats;
Group G: was a control group of not castrated rats

The drugs were dissolved in oil before inoculation. The days the groups got combined treatment of 2 drugs this was done separately at two different inoculation points. All treatments were started one week after castration.

After 15 days of treatment 8 rats in each group were sacrificed. In all animals were determined:
- weight and size of uterus
- blood plasma levels of LH
- hypophysis levels of LH

After 30 days the rest of the rats were sacrificed and the same data was determined. The data was determined using standard methods. All data were statistically analyzed by standard methods.

### Results:

The relative weight of uterus (g uterus/Kg rat weight) alter 15 days were approximately equal in all groups except the castrated control, which had a significantly lower uterus weight. At 30 days the results were similar. The castrated control group still had the lowest weight. However, here the group only getting tibolone had a uterus weight that was some grams lower than the rest.

With respect to the plasma LH levels, the tibolone only and castrated control group had the highest levels. This was both at 15 and 30 days. However, at 30 days the tibolone only group had LH levels below the castrated control group.

With respect to hypophysis levels of LH, after 30 days treatment the highest levels were observed in the tibolone only and castrated control group.

### Discussion:

From the uterus weight data one may deduce that the combination estrogen/tibolone has an estrogen / progestin effect similar to the known estrogen/noretisterone standard.

The plasmatic LH data demonstrates the fundamental estrogen action on feed back regulation of hypophysis secretion. All estrogen groups had levels corresponding to the not castrated control group.

The hypophysis data demonstrates the progestin effect of the tibolone/estrogen combination. At 30 days the hypophysis LH levels of the tibolone/estrogen groups were similar to the not castrated control group.

## Claims

1. A pharmaceutical product comprising an effective amount of tibolone or derivative thereof, an effective amount of estradiol or derivative thereof and a pharmaceutically acceptable carrier, wherein the product is provided as a combined preparation for simultaneous, separate or sequential use in a method for hormone replacement therapy or prevention of hypoestrogenism associated clinical symptoms in a human person.

2. The pharmaceutical product of claim 1, wherein the pharmaceutical product is provided as:
- first container comprising a composition comprising an effective amount of tibolone or derivative thereof and a pharmaceutically acceptable carrier;
- second container comprising a composition comprising an effective amount of estradiol or derivative thereof and a pharmaceutically acceptable carrier; and
- instructions for combined sequential and/or simultaneous administration of the compositions of first and second container for simultaneous or sequential use in a method for hormone replacement therapy or prevention of hypoestrogenism associated clinical symptoms in a human person.

3. The pharmaceutical product of claim 1, wherein the pharmaceutical product is a pharmaceutical composition comprising an effective amount of tibolone or derivative thereof, an effective amount of estradiol or derivative thereof and a pharmaceutically acceptable carrier for hormone replacement therapy or prevention of hypoestrogenism associated clinical symptoms in a human person.

4. The pharmaceutical product of any of claims 1 to 3, wherein the pharmaceutical product comprises a composition comprising from 50 µg to 5mg of tibolone or derivative thereof and a composition comprising from 50 µg to 5mg of estradiol or derivative thereof.

5. The pharmaceutical product of any of claims 1 to 4, wherein the pharmaceutical product comprises tibolone and β-estradiol.

6. The pharmaceutical product of any of claims 1 to 5, wherein the human is a postmenopausal woman.

7. Use of a pharmaceutical product, of any of the preceding claims, for the manufacture of a medicament for hormone replacement therapy or prevention of hypoestrogenism associated clinical symptoms in a human person.

8. The use of claim 7, wherein the human is a postmenopausal woman.

9. The use of any of claims 7 to 8, wherein the tibolone or derivative thereof is administered at a dosage from 5 µg/kg to 5mg/kg and the estradiol or derivative thereof is administered at a dosage from 5 µg/kg to 5mg/kg.

10. The use of any of claims 8 to 9, wherein the clinical symptoms of a postmenopausal woman are osteoporosis, increased cholesterol levels that lead to atherosclerosis, depression, headaches, nausea or climacteric symptoms, and wherein the pharmaceutical product is administered orally to the woman.
